# EUROPEAN PATENT APPLICATION

(11) **EP 3 124 049 A1**
(43) Date of publication of application: **01.02.2017**
(21) Application number: 15770023.8
(22) Date of filing: 27.03.2015
(51) Int. Cl.: A61K 47/32, A61K 9/06, A61K 9/107, A61K 31/573, A61K 47/14, A61K 47/34, A61K 47/44, A61P 17/00, A61P 37/08

(54) **PROCESS FOR PRODUCING COMPOSITION OF EMULSION PREPARATION TYPE**

(30) Priority: 27.03.2014 JP 2014082315; 27.03.2014 JP 2014082316; 01.04.2014 JP 2014084984
(71) Applicant: Pola Pharma Inc., Shinagawa-ku Tokyo 141-0031 (JP)
(72) Inventor: MASUDA, Takaaki, Yokohama-shi Kanagawa 244-0812 (JP); KOBAYASHI, Hirokazu, Yokohama-shi Kanagawa 244-0812 (JP)
(74) Representative: dompatent von Kreisler Selting Werner - Partnerschaft von Patent- und Rechtsanwälten mbB
(86) International application number: PCT/JP2015/059763
(87) International publication number: WO 2015/147296

(57) **Abstract**

Provided is a technique for preparing a composition which after application forms a coating film having excellent properties although the recipe is the same. The technique is a process 1) for producing a composition of the oil-in-water emulsion preparation type which contains an optionally alkyl-modified carboxyvinyl polymer and/or a salt thereof and further contains a nonionic surfactant. The process is characterized by preparing an oil-in-water emulsion at a temperature higher than the cloud point of the nonionic surfactant, cooling the emulsion to or below the cloud point of the nonionic surfactant, then adding a carboxyvinyl polymer, and thereafter neutralizing the carboxyvinyl polymer with an alkali agent.

## Description

### Technical Field

The present invention relates to a method of producing a composition, further specifically, relates to a method of producing a composition of an emulsifier type.

### Background Art

When a carboxyvinyl polymer is neutralized with an alkali, a cross-linked structure is generated between carboxyl groups owing to van der Waals' force, to form a thixotropic strong gel. The strong structure of this carboxyvinyl polymer has been utilized for stabilization of an oil-in-water emulsifier type. As the oil-in-water emulsifier type using such a carboxyvinyl polymer, emulsifier types using a fatty acid soap for stabilizing a cross-linked structure are often used. In this case, an aqueous phase containing a carboxyvinyl polymer is added to an oil phase at a temperature of 80 to 905ºC, subsequently, an alkaline agent to neutralize the carboxyvinyl polymer is added to achieve neutralization thereof, and thereafter, the mixture is cooled, to obtain an emulsified composition, in an ordinary method. This contains a hydrophilic nonionic surfactant in addition to a soap in many cases, because in emulsification using the hydrophilic nonionic surfactant, hydrophilicity of the hydrophilic nonionic surfactant is lowered at a temperature of the cloud point or higher, to attain uniform distribution into an oil phase. In emulsification at a temperature of the cloud point or lower, distribution of the nonionic surfactant into an oil droplet possibly becomes non-uniform. In contrast, stabilization of an oil droplet by an aqueous phase is easily obtained in emulsification at a temperature of the cloud point or lower, thus, in emulsification using an ingredient (carboxy compound) having the Krafft point such as a soap, an alkyl-modified carboxyvinyl polymer and the like, there is also a case of adopting emulsification at a temperature lower than the cloud point of a nonionic surfactant and higher than the Krafft point of a carboxy compound (see, e.g., Patent document 1, Patent document 2, Patent document 3, Patent document 4).

Meanwhile, a production method including a mixing step at a low temperature range in an emulsification method using a carboxyvinyl polymer is tried in an emulsification method via a liquid crystal (see, e.g., Patent document 5).

### Prior Art Document

### Patent Document

Patent document 1: JP-A No. 2006-8709
Patent document 2: JP-A No. 2005-89366
Patent document 3: Japanese Patent Application National Publication No. 9-512277
Patent document 4: JP-A No. 2004-43785
Patent document 5: JP-A No. 10-180085

### Summary of the Invention

### Problems to be Solved by the Invention

A production method of a pharmaceutical composition of an emulsifier type containing a carboxyvinyl polymer in which a carboxyvinyl polymer is added in a low temperature range and neutralization is performed is not known. It is also not known at all that an emulsion prepared by such an emulsification method forms an excellent coating film after application to skin. Further, it is also not known at all that an external preparation of the same formulation forms a coating film having an entirely different property. Further, a phenomenon that a coating film having a different property is formed depending on a difference of the production method though the formulation is the same is also not known. Further, to make a scarcely destructible strong moisture-retaining film on skin is preferable also for absorption and orientation of an active ingredient. However, a method of making a film having such a property is not known under current state though such a method is required. It can also be said that, in other words, a technology for changing the physical properties of a composition of an emulsifier type without changing the formulation, that is, for enhancing the efficacy of an active ingredient depending on the production method is required.

The present invention has been accomplished under such conditions and has an object of providing a technology for preparing a composition which forms a coating film having an excellent property after application though the formulation is the same.

### Means for Solving the Problem

In view of such conditions, the present inventors have intensively studied and stepped up efforts in search of a technology of preparing a composition of an emulsifier type, which forms a coating film having excellent properties after application though the formulation is the same, and resultantly found that the physical properties of a composition can be improved by 1) a method of producing a composition of an oil-in-water emulsifier type containing a carboxyvinyl polymer optionally alkyl-modified and/or a salt thereof and a nonionic surfactant, wherein an oil-in-water emulsion is prepared at a temperature higher than the cloud point of the above-described nonionic surfactant, the material is cooled to a temperature not higher than the cloud point of the nonionic surfactant, then, a carboxyvinyl polymer is added, and thereafter, the carboxyvinyl polymer is neutralized with an alkaline agent, leading to completion of the invention. That is, the present invention is as described below.
<1> 1) A method of producing a composition of an oil-in-water emulsifier type containing a carboxyvinyl polymer optionally alkyl-modified and/or a salt thereof (in the present specification, this concept is referred to simply as "carboxyvinyl polymer optionally alkyl-modified" or "carboxyvinyl polymer" in some cases) and a nonionic surfactant, wherein an oil-in-water emulsion is prepared at a temperature higher than the cloud point of the above-described nonionic surfactant, the emulsion is cooled to a temperature not higher than the cloud point of the nonionic surfactant, then, a carboxyvinyl polymer is added, and thereafter, the carboxyvinyl polymer is neutralized with an alkaline agent.
<2> The production method of the composition according to <1>, wherein the above-described temperature higher than the cloud point is 75 to 90°C.
<3> The production method according to <1> or <2>, wherein the above-described temperature not higher than the cloud point is 25 to 35°C.
<4> The production method of the composition according to any one of <1> to <3>, wherein the above-described nonionic surfactant is selected from a polyoxyethylene alkyl ether, castor oil optionally polyoxyethylene-hardened and a fatty acid monoglyceride.
<5> The production method of the composition according to any one of <1> to <4>, wherein the surfactant is only a nonionic surfactant.
<6> The production method of the composition according to any one of <1> to <5>, wherein the above-described composition contains one selected from a moisturizer, an antihistamine agent, a non-steroidal anti-inflammatory agent, a steroid, a bactericide, an anti-fungal agent, an antibiotic, an anti-virus agent, an immunosuppressive agent and a vitamin, as an active ingredient.
<7> A composition comprising a carboxyvinyl polymer optionally alkyl-modified and/or a salt thereof and a nonionic surfactant, wherein the residual viscosity measured by a cone-plate viscometer under 32°C homeothermic condition is 200 mPa·s or less and the Casson yield value thereof is 40000 mPa or more.
<8> A skin external preparation having an emulsifier type which has an aqueous phase in the outermost phase and having a viscosity at 25°C of 1000 to 3000 mPa·s, wherein the variance of the measured value of the spread diameter measured by using a spread meter depending on temperature change is a value of 1 to 4.

### Effect of the Invention

The present invention can provide a technology for preparing a composition which forms a coating film having excellent properties after application though the formulation is the same.

### Brief Description of Drawings

Fig. 1 is a view showing emulsified particles directly after preparation (drawing substitute photograph).
Fig. 2 is a view showing the results of a 80°C stability test (drawing substitute photograph).

### Modes for Carrying Out the Invention

### <1> Essential constituent element of composition according to production method of present invention

It is an essential constituent element for the composition according to the production method of the present invention that the composition comprises 1) a carboxyvinyl polymer optionally alkyl-modified and/or a salt thereof (in the present specification, this concept is referred to simply as "carboxyvinyl polymer optionally alkyl-modified" or "carboxyvinyl polymer" in some cases), 2) a nonionic surfactant and 3) an alkaline agent to neutralize the carboxyvinyl polymer, as an essential ingredient, and the composition is an emulsifier type.

As the above-described carboxyvinyl polymer optionally alkyl-modified, alkyl-modified carboxyvinyl polymers obtained by introducing a long-chain alkyl group such as "Pemulen TR-1", "Pemulen TR-2" or "Carbopol 1382" (all are manufactured by Lubrizol Advanced Materials) and the like can also be used, in addition to usual carboxyvinyl polymers. As the above-described long-chain alkyl group, those having a number of carbon atoms of 10 to 30 are preferable. The carboxyvinyl polymer optionally alkyl-modified is neutralized with an alkaline agent described later and operates as a thickener, and has an action of stabilizing an emulsification system and reinforcing a coating film formed after application of a composition. For performing such an effect, it is preferable that the carboxyvinyl polymer optionally alkyl-modified and/or the salt thereof is contained in a total amount of 0.3 to 1.2% by mass, further preferably 0.5 to 1.0% by mass. The reason for this is that preferable stability is manifested at amounts in this range. As the above-described carboxyvinyl polymer, a carboxyvinyl polymer which a 0.2% aqueous solution thereof shows a viscosity of 2500 to 30000 mPa·s at 20°C in a neutral region is preferably used.

For thickening the above-described carboxyvinyl polymer, an alkaline agent to neutralize the carboxyvinyl polymer is contained in the above-described composition. As the above-described alkaline agent, an organic amine is preferable, and for example, triethanolamine, triethylamine, monoethanolamine, isopropanolamine and the like are suitably exemplified. Of them, isopropanolamine is particularly preferable. It is preferable that such an organic amine is contained in the above-described composition, in an amount of 0.05 to 1.5% by mass, preferably 0.05 to 1.0% by mass, further preferably 0.05 to 0.8% by mass, particularly preferably 0.08 to 0.5% by mass. The reason for this is that preferable viscosity is manifested at amounts in this range. Here, an alkaline agent aqueous solution can be added so that pH of the composition of the present invention is preferably 4 to 8. The lower limit of pH of the composition of the present invention is preferably 4, further preferably 4.5. Meanwhile, the upper limit of pH of the composition of the present invention is preferably 6, further preferably 5.5.

The above-described composition is characterized by containing a nonionic surfactant. As the nonionic surfactant, monoglycerides such as stearic acid monoglyceride, oleic acid monoglyceride and the like, sorbitan fatty acid esters such as sorbitan stearic acid ester, sorbitan oleic acid ester and the like, ester type nonionic surfactants such as POE stearic acid ester, POE oleic acid ester and the like, castor oils optionally POE-hardened, POE sorbitan fatty acid esters such as POE sorbitan oleic acid ester, POE sorbitan stearic acid ester and the like, ether type nonionic surfactants such as POE oleyl ether, POE cetyl ether and the like, etc. are suitably exemplified, and as the hydrophilic nonionic surfactant, POE-hardened castor oils and/or ether type nonionic surfactants are preferably exemplified, and a form containing both of them is particularly preferable. As the lipophilic surfactant, fatty acid monoglycerides are preferably exemplified. It is preferable to use substantially only a nonionic surfactant, as the surfactant, in the above-described composition. The reason for this is that the cloud point can be guessed definitely. It is preferable that the content of the above-described surfactant is 3 to 7% by mass, preferably 3 to 5% by mass in total.

Arbitrary ingredients usually used in a skin external preparation can be contained, in addition to the above-described ingredients, in the above-described composition. As such arbitrary ingredients, for example, hydrocarbons such as squalane, vaseline and the like, ester oil solutions such as jojoba oil, cetyl isooctanoate, isopropyl myristate and the like, olive oil, triglycerides such as medium chain fatty acid triglycerides, polyhydric alcohols such as 1,3-butanediol, propylene glycol, glycerin and polyethylene glycol, thickeners such as xanthan gum optionally alkyl-modified and the like, fatty acids such as stearic acid, myristoleic acid, myristic acid, lauric acid and the like or salts thereof, higher alcohols such as cetostearyl alcohol, behenyl alcohol, isostearyl alcohol, oleyl alcohol and the like, etc. are suitably exemplified. Of them, fatty acids impair a cross-linked structure of a carboxyvinyl polymer in some cases, thus, a form containing substantially no fatty acid is preferable. As the dosage form, an emulsified form having an aqueous phase as the outer phase is preferable, and such dosage forms are collectively called an oil-in-water emulsifier type. The dispersed droplet may be an oil droplet or an emulsion.

The above-described composition can be a pharmaceutical composition, preferably an external pharmaceutical composition, by containing active ingredients as a medicinal product. As the active ingredient, for example, those selected from a moisturizer, an antihistamine agent, a non-steroidal anti-inflammatory agent, a steroid, a bactericide, an anti-fungal agent, an antibiotic, an immunosuppressive agent and vitamins are preferably exemplified. As the moisturizer, heparinoid or urea and the like are preferably exemplified, as the antihistamine agent, for example, olopatadine, diphenhydramine and the like are suitably exemplified, as the non-steroidal anti-inflammatory agent, for example, indometacin, suprofen, ketoprofen, ketotifen and the like are suitably exemplified, as the steroid, hydrocortisone, dexamethasone, prednisolone, clobetasone, clobetasol propionate, betamethasone butyrate propionate, beclomethasone propionate, mometasone furoate, clobetasone butyrate and esters thereof and the like are suitably exemplified, as the bactericide, povidone iodine, benzalkonium chloride, chlorhexidine gluconate and the like are preferably exemplified, as the anti-fungal agent, terbinafine, butenafine, bifonazole, luliconazole and the like are suitably exemplified, as the antibiotic, achromycin, gentamicin, penicillin and the like are suitably exemplified, as the anti-virus agent, acyclovir, adenine arabinoside and the like are suitably exemplified, as the immunosuppressive agent, for example, cyclophosphamide, cyclosporine, tacrolimus and the like are suitably exemplified, as the vitamins, vitamin A such as retinol, retinoic acid, adapalene, tretinoin tocoferil and the like, vitamin D such as calciferol, maxacalcitol and the like, vitamin E such as tocopherol and the like, vitamin B such as vitamin B₁₂ or vitamin B₆, vitamin C such as ascorbic acid and ascorbic acid glucoside, etc. are suitably exemplified. Of them, poor solubility active ingredients are particularly preferable. The reason for this is that orientation to an affected site is enhanced by containing such ingredients in the system. The contents thereof are approximately 0.01 to 10% by mass though varying depending on each drug efficacy and acting dosage.

### <2> Production method of composition of present invention

The method of producing a composition of the present invention is characterized in that an oil-in-water emulsion is prepared at a temperature higher than the cloud point of a nonionic surfactant, the emulsion is cooled to a temperature not higher than the cloud point of a nonionic surfactant, then, a carboxyvinyl polymer optionally alkyl-modified is added, and thereafter, the carboxyvinyl polymer optionally alkyl-modified is neutralized with an alkaline agent. When a plurality of nonionic surfactants are present, the highest cloud point temperature is preferably adopted, for the temperature higher than the cloud point of a nonionic surfactant, and temperatures of roughly 75 to 90°C are applied. When a plurality of nonionic surfactants are present, the lowest cloud point temperature is preferably adopted, for the temperature not higher than the cloud point of a nonionic surfactant, and temperature of roughly 20 to 65°C are preferable, temperature of 25 to 50°C are more preferable, temperature of 25 to 35°C are further preferable. It is preferable that the carboxyvinyl polymer optionally alkyl-modified is dissolved at a water addition amount near the lowest limit for showing flowability and the solution is added to the composition, and it is specifically preferable that the polymer is dissolved in 5 to 65% by mass of water and the solution is added to the composition. It is desirable that such water amount is 35 to 60% by mass with respect to the whole composition. It is preferable that the alkaline agent to be added later is diluted with water to an extent not disturbing dispersion and the solution is added to the composition, and it is specifically preferable that the alkaline agent is diluted with 1 to 5% by mass of water and the solution is added to the composition. It is preferable that the residual water is added as an aqueous phase for preparing an oil-in-water emulsion. The preparation procedure will be illustrated in divided steps below.

### <Step 1>

A carboxyvinyl polymer optionally alkyl-modified is dissolved in a small amount of water previously, to prepare a solution of the carboxyvinyl polymer optionally alkyl-modified. According to the same manner, an alkaline agent aqueous solution is prepared. These two kinds of solutions are regulated at respective temperatures of not higher than the cloud point of a nonionic surfactant to be added.

### <Step 2>

The residual water, and aqueous ingredients, for example, a polyhydric alcohol and water-soluble additives are combined, and regulated at a temperature higher than the cloud point as the emulsification temperature in advance. Concurrently, oily ingredients including a nonionic surfactant are combined, and regulated at a temperature higher than the cloud point as the emulsification temperature in advance.

### <Step 3>

To the aqueous phase regulated at a temperature higher than the cloud point of a nonionic surfactant is added gradually the oil phase regulated at a temperature higher than the cloud point in like manner under stirring, to prepare an oil-in-water emulsion, and this is cooled with stirring, and cooled down to the cloud point or lower. When a temperature of the cloud point or lower is attained, the solution of the carboxyvinyl polymer optionally alkyl-modified is added gradually under stirring. After addition, the mixture is stirred until homogeneity, and thereafter, the alkaline agent aqueous solution is added gradually, to obtain the composition of the present invention.

Here, the alkaline agent aqueous solution can be added so that pH of the composition of the present invention is preferably 4 to 8. The lower limit of pH of the composition of the present invention is preferably 4, further preferably 4.5. Meanwhile, the upper limit of pH of the composition of the present invention is preferably 6, further preferably 5.5.

Thus obtained composition is a composition revealing little temperature gradient of viscosity since the thickened cross-linked structure of the carboxyvinyl polymer optionally alkyl-modified is scarcely affected by a lowering of surface active action due to the cloud point of a nonionic surfactant, thus, the composition becomes a system which is stable even at higher temperatures with a small amount of a thickener. When the composition is applied to form a coating film, it is a coating film excellent in the action of suppressing TEWL. As the characteristic of such a coating film, it has an action of enhancing percutaneous absorption of an active ingredient owing to the occlusion effect on skin. Further, when attention is paid to the emulsion, emulsion particles are fine and uniform since the total amount of the nonionic surfactant blended is effectively used for emulsification. Additionally, since the cross-linked structure of the carboxyvinyl polymer optionally alkyl-modified is not broken, a strong gel structure is made.

The production method of the present invention is suitable for production of a skin external preparation, particularly, of an external pharmaceutical composition.

The composition obtained by the production method of the present invention preferably has physical properties as described below. The composition having such physical properties is novel per se.

The composition of the present invention is characterized by a fact that it is a preparation showing low residual viscosity, while showing high Casson yield value when the rheology is examined at condition near use mode for applying to skin, for example, at a temperature of 32°C. Therefore, the composition of the present invention is characterized by a fact that it is a preparation showing little change of rheology properties from initiation of use until completion of use and excellent in stability in preservation. By having such characteristics, physical load in spreading can be reduced even on sensitive skin in its use. By this reason, transient stimulus expression can be suppressed on sensitive skin. Therefore, the composition of the present invention can be administered as an external pharmaceutical composition for treatment of atopic dermatitis and the like. Such physical properties are specifically illustrated as described below.
(1) The residual viscosity measured under 32°C homeothermic condition by a cone-plate viscometer is 200 mPa· s or less, more preferably 190 mPa·s or less, and further preferably, besides, 100 mPa·s or more, and 120 mPa·s or more and the Casson yield value is 40000 mPa or more, more preferably 42000 mPa or more, besides, 60000 mPa or less, more preferably 50000 mPa or less.
(2) It is preferable for the composition of the present invention that the square root of shear rate shows good linearity in a range of 1 to 15. That is, in the Casson plot, the value of the square of correlation coefficient is preferably 0.98 or more in a range of the square root of shear rate of 1 to 15. In a usual composition, structural change tends to occur because of hydrophilic balance of a thickener constituting the structure and the effect of the cloud point of a surfactant exerting an influence on the cross-linked structure and the like. In a preferable form of the composition of the present invention, the above-described structural change poorly occurs since change of viscoelasticity shows excellent linearity even in the above-described range.
(3) For this reason, the composition of the present invention has a characteristic that change of a feeling of use such as spreadability and the like is extremely small from initiation of use until completion of use in use thereof. It is guessed that, by this characteristic, the composition imparts low physical stimulus to even sensitive skin.
(4) The viscosity at 25°C of the composition of the present invention measured by a cone-plate viscometer is preferably 1300 mPa·s or more, more preferably 1400 mPa·s or more, further preferably 1500 Pa·s or more, particularly preferably 1600 mPa·s or more, besides, preferably 3000 mPa· s or less, more preferably 2000 mPa·s or less, further preferably 1900 mPa·s or less.

The skin external preparation of the present invention is a skin external preparation having an emulsifier type having an aqueous phase in the outermost phase, and having a viscosity at 25°C of 1000 to 3000 mPa·s, preferably 1500 to 2500 mPa· s, and the variance of the measured value of the spread diameter measured by using a spread meter depending on temperature change is a value of 1 to 4, preferably 1. 2 to 3. 6. The variance can be determined by the following method. Such a skin external preparation is excellent in usability.

That is, the spread areas at at least two temperatures of the skin external preparation are measured as the spread diameter by using a spread meter (hereinafter, the measured value is referred to as "spread meter diameter" in some cases) . When the variance of the measured value depending on temperature change is determined and the variance shows a value of 1 to 4, the above-described skin external preparation can be discriminated to be excellent in usability, namely, this method can be applied to a method for evaluating and discriminating a skin external preparation. Usually, in the case of a skin external preparation of an emulsifier type, it is necessary to keep viscosity at around 3000 mPa·s at 25°C so that emulsified particles are not easily coalesced, for providing stability. It is usual that an emulsion manifesting a viscosity of around 3000 mPa·s at 25°C shows typically a viscosity around 5000 mPa·s at 5°C and a viscosity around 1000 mPa·s at 40°C. The reason is guessed that the viscous structure is ascribable to intermolecular affinity and the emulsified structure of waxes. Therefore, even a emulsion showing suitable spreadability at about 32°C shows entirely different spreadability and the feeling of use varies remarkably at 5°C or 40°C. The evaluation method of the present invention is characterized in that the temperature dependency of this feeling of use is distinguished and one showing little change of usability depending on temperature is evaluated as a good preparation. By conducting such evaluation, a skin external preparation showing constant usability irrespective of temperature can be selected, and a preparation manifesting a feeling of stimulation by application at lower temperatures can be avoided. This is advantageous since in a skin external preparation for anti-inflammation, administration of a skin external preparation leads directly to symptomatic improvement. In other words, a skin external preparation for anti-inflammation can be administered without considering use conditions. The emulsifier type having an aqueous phase in the outermost phase means an emulsion in which oil droplets or water-in-oil emulsified droplets are dispersed in the aqueous phase.

Therefore, in determining variance, at least temperatures around 25°C as usual use temperature and temperatures around 5°C which is a low temperature at which spreadability is disturbed are preferably used as the at least two temperatures, and it is preferable that also temperatures around 32°C close to the temperature of the applied surface are used additionally. The spread meter diameters at these temperatures are calculated, the average spread meter diameter at each temperature is calculated, and the variance value between the average spread meter diameters at respective temperatures is determined. Instead of the variance value, the standard deviation as the square root of the variance value can also be used. When these values are 1 to 4, further preferably 1.2 to 3. 6 in terms of the variance value, usability of the skin external preparation can be discriminated and evaluated as good, thus, it is preferable that the skin external preparation of the present invention has such physical properties.

pH of the composition of the present invention is preferably 4 to 8. The lower limit of pH of the composition of the present invention is preferably 4, further preferably 4.5. The upper value of pH of the composition of the present invention is preferably 6, further preferably 5.5.

It is preferable that the composition of the present invention is a skin external preparation, particularly, a skin external medicine.

A preferable form of the composition of the present invention is a composition having an emulsifier type having an aqueous phase in the outermost phase and having a viscosity at 25°C of 1000 to 3000 mPa·s, more preferably 1500 to 2500 mPa·s, and the variance of the measured value of the spread diameter measured by using a spread meter depending on temperature change is preferably a value of 1 to 4, further preferably 1.2 to 3.6. The variance can be determined by the above-described method.

Such a composition shows constant usability irrespective of temperature and can avoid manifestation of a feeling of stimulation by application at lower temperatures. This is advantageous since in a skin external preparation for anti-inflammation, administration of a skin external preparation leads directly to symptomatic improvement. In other words, such a skin external preparation can be administered to skin for anti-inflammation without considering use conditions. The emulsifier type having an aqueous phase in the outermost phase means an emulsion in which oil droplets or water-in-oil emulsified droplets are dispersed in the aqueous phase.

The skin external preparation of the present invention has its value of preferably 1 to 4, further preferably 1.2 to 3.6 in terms of the variance value.

The present invention will be described further in detail by examples shown below.

### Example 1

A pharmaceutical composition 1 was prepared by the production method of the present invention according to a formulation shown below. That is, ingredients (a), (b), (c) and (d) were weighed, and of them, (c) and (d) were mixed and dissolved with stirring at room temperature and (a) and (b) were mixed and dissolved with stirring at 75°C, and kept at respective dissolution temperatures. Under stirring, to (b) was added (a) gradually and emulsification was caused, and the emulsion was cooled with stirring and when cooled down to 30°C, (c) was added gradually under stirring, and when a uniform mixture was obtained, further, (d) was added gradually and neutralization was accomplished, to obtain a pharmaceutical composition 1 of the present invention. pH of the pharmaceutical composition 1 was 4.5.

**[Table 1]**

| Ingredient | Content (% by mass) |
|---|---|
| (a) | |
| Clobetasone butyrate | 0.5 |
| Squalane | 2 |
| White vaseline | 9 |
| Diisopropyl adipate | 4 |
| Cetanol | 1 |
| Glycerin monostearate | 2 |
| POE(60) hydrogenated castor oil | 1.5 |
| POE(23) cetyl ether | 2 |
| Medium chain fatty acid triglyceride | 6 |
| (b) | |
| 1,3-butanediol | 14 |
| Sodium edetate | 0.1 |
| Water | 45 |
| (c) | |
| Carboxyvinyl polymer | 0.7 |
| Water | 10 |
| (d) | |
| Diisopropanolamine | 0.1 |
| Water | 2.1 |

### <Comparative Example 1>

A pharmaceutical composition of Comparative Example 1 was produced by using the same formulation ingredients as those of the pharmaceutical composition 1 of the present invention and by changing only the production method. That is, (a), (b), (c) shown in Table 2 were weighed, and of them, (a) and (b) were mixed and dissolved with stirring at 75°C and (c) was mixed and dissolved with stirring at room temperature, and kept at respective dissolution temperatures. Under stirring, to (b) was added (a) gradually and emulsification was caused, and when a uniform mixture was obtained, the mixture was cooled with stirring, and when cooled down to 30°C, further, (c) was added gradually and neutralization was accomplished, to obtain a pharmaceutical composition of Comparative Example 1. pH of the pharmaceutical composition of Comparative Example 1 was 4.5.

**[Table 2]**

| Ingredient | Content (% by mass) |
|---|---|
| (a) | |
| Clobetasone butyrate | 0.5 |
| Squalane | 2 |
| White vaseline | 9 |
| Diisopropyl adipate | 4 |
| Cetanol | 1 |
| Glycerin monostearate | 2 |
| POE(60) hydrogenated castor oil | 1.5 |
| POE(23) cetyl ether | 2 |
| Medium chain fatty acid triglyceride | 6 |
| (b) | |
| 1,3-butanediol | 14 |
| Sodium edetate | 0.1 |
| Water | 55 |
| Carboxyvinyl polymer | 0.7 |
| (c) | |
| Diisopropanolamine | 0.1 |
| Water | 2.1 |

### <Comparison of physical properties>

The physical properties were compared between the pharmaceutical composition 1 of the present invention and the pharmaceutical composition of Comparative Example 1. The physical property values examined were emulsified particles, viscosity, stability at 80°C, and the residual viscosity and the Casson yield value determined from the Casson plot.

### (1) Emulsified particles

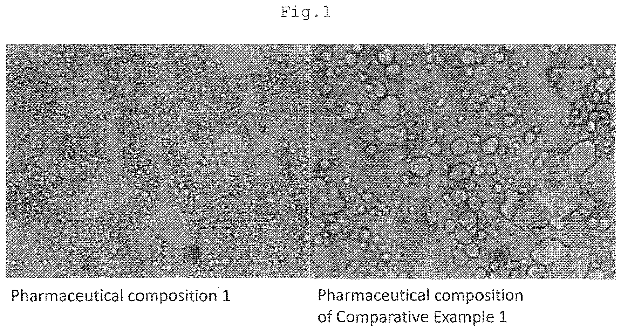

Micrographs of emulsified particles of the pharmaceutical composition 1 of the present invention and the pharmaceutical composition of Comparative Example 1 are shown in Fig. 1. It is understood from this that the pharmaceutical composition of the present invention is composed of fine and uniform emulsified particles, while the pharmaceutical composition of Comparative Example is composed of non-uniform and large emulsified particles.

### (2) Viscosity

The pharmaceutical composition 1 of the present invention and the pharmaceutical composition of Comparative Example 1 were measured by a cone-plate viscometer (apparatus model name: RE-80R, name of manufacturing company: TOKI SANGYO Co., Ltd., conditions: rotor: 3°×R14, measurement temperature: 25°C, rotation frequency: 50 rpm, measurement time: 3 minutes). As a result, the pharmaceutical composition 1 of the present invention had a viscosity of 1733 mPa·s, and the pharmaceutical composition of Comparative Example 1 had a viscosity of 1133 mPa·s. It is understood that in the pharmaceutical composition 1 of the present invention, the cross-linked structure of a carboxyvinyl polymer is not destructed by a nonionic surfactant and higher viscosity is obtained. In other words, this is also understood that in the pharmaceutical composition of the present invention, a strong emulsified interface is formed since a large portion of a nonionic surfactant is correlated with emulsification.

### (3) Stability at 80°C

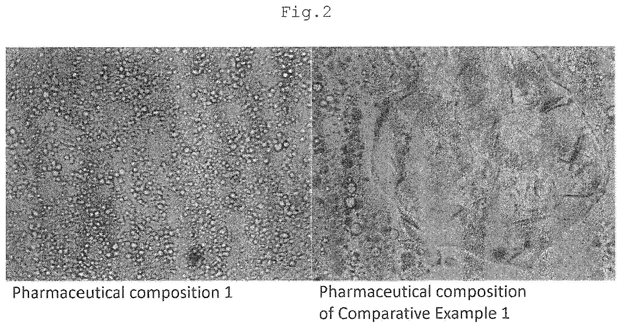

The pharmaceutical composition 1 of the present invention and the pharmaceutical composition of Comparative Example 1 were preserved at 80°C for 6 hours, and the emulsified conditions were microscopically and macroscopically observed. A micrograph of the pharmaceutical composition 1 preserved at 80°C for 6 hours is shown in Fig. 2. It is understood from this that in the pharmaceutical composition 1 of the present invention, change of emulsified particles was not observed even under this preservation condition. In contrast, it is understood that in the pharmaceutical composition of Comparative Example 1, particles are coalesed and roughened. In macroscopic observation, no change was found in the pharmaceutical composition 1 of the present invention, while in the pharmaceutical composition of Comparative Example 1, separation into two phases was recognized.

### (4) Residual viscosity and Casson yield value

For the pharmaceutical composition 1 of the present invention and the pharmaceutical composition of Comparative Example 1, shear stress (S) against a shear rate (D) of 2 to 200 (1/s) was measured using a cone-plate viscometer (apparatus model name: RE-80R, name of manufacturing company: TOKI SANGYO Co., Ltd., conditions: rotor: 3°×R14, measurement temperature: 32°C, rotation frequency: 1, 2.5, 5, 10, 20, 50 and 100 rpm, measurement time: 3 minutes), and the residual viscosity was calculated from the square of a gradient a in the relational equation; √S = a√D+b (a and b are a coefficient) and the Casson yield value was calculated from the square of an intercept b thereof. The results are shown in Table 3. It is guessed from this that the pharmaceutical composition 1 produced by the production method of the present invention shows good spread in spreading since the residual viscosity is low and manifests high stability in preservation and high coating film strength after application since the Casson yield value is high.

**[Table 3]**

| Sample | Residual viscosity (mPa·s) | Casson yield value (mPa) |
|---|---|---|
| Pharmaceutical composition 1 of the present invention | 183 | 46376 |
| Pharmaceutical composition of Comparative Example 1 | 236 | 24661 |

### (5) Variance of spread meter diameter

For the pharmaceutical composition 1, the spread meter diameters at 5°C, 25°C and 32°C were measured using a spread meter (apparatus model name: type IMC-15E2, name of manufacturing company: Imoto Machinery Co., Ltd., conditions: sample bore diameter: 10 mm, sample bore depth: 6.37 mm, mass of load plate: 115 g, measurement time: 60 seconds). The measurement was repeated three times, and the average and the standard deviation were calculated to find that the value was 35.59 ± 0.38 mm at 5°C, 36.60 ± 0.36 mm at 25°C and 38.32 ± 0.44 mm at 32°C, and the standard deviation of the three averages was 1.38, and the variance thereof was 1.9.

For the pharmaceutical composition 1, usability was confirmed to find that a transient stimulus by the composition was not felt even on sensitive skin.

In contrast, usability was confirmed for the pharmaceutical composition of Comparative Example 1 to find that a transient stimulus by the composition was manifested on sensitive skin.

### Example 2

A skin external preparation 2 (containing no active ingredient) was produced by the production method described above according to a formulation show below. That is, ingredients (a), (b), (c) and (d) were weighed, and of them, (c) and (d) were mixed and dissolved with stirring at room temperature and (a) and (b) were mixed and dissolved with stirring at 75°C, and preserved at respective dissolution temperatures. Under stirring, to (b) was added (a) gradually and emulsification was caused, and the emulsion was cooled with stirring, and when cooled down to 30°C, (c) was added gradually under stirring, and when a uniform mixture was obtained, further, (d) was added gradually and neutralization was accomplished, to obtain a skin external preparation 2. pH of the skin external preparation 2 was 4.5.

**[Table 4]**

| Ingredient | Content (% by mass) |
|---|---|
| (a) | |
| Squalane | 2 |
| White vaseline | 9 |
| Diisopropyl adipate | 4 |
| Cetanol | 1 |
| Glycerin monostearate | 2 |
| POE(60) hydrogenated castor oil | 1.5 |
| POE(23) cetyl ether | 2 |
| Medium chain fatty acid triglyceride | 6 |
| (b) | |
| 1,3-butanediol | 14 |
| Sodium edetate | 0.1 |
| Water | 45.5 |
| (c) | |
| Carboxyvinyl polymer | 0.7 |
| Water | 10 |
| (d) | |
| Diisopropanolamine | 0.1 |
| Water | 2.1 |

The skin external preparation 2 had a residual viscosity of 123 mPa·s and a Casson yield value of 44642 mPa.

### <Comparative Example 2>

A skin external preparation of Comparative Example 2 was produced by using the same formulation ingredients as those of the skin external preparation 2 and by changing only the production method. That is, (a), (b) and (c) shown in Table 5 were weighed, and of them, (a) and (b) were mixed and dissolved with stirring at 75°C and (c) was mixed and dissolved with stirring at room temperature, and preserved at respective dissolution temperatures. Under stirring, to (b) was added (a) gradually and emulsification was caused, and when a uniform emulsion was obtained, the emulsion was cooled with stirring, and when cooled down to 30°C, further, (c) was added gradually and neutralization was accomplished, to obtain a skin external preparation of Comparative Example 2. pH of the medicinal composition of Comparative Example 2 was 4.5. The residual viscosity and the Casson yield value of the medicinal composition of Comparative Example 2 were measured to find that the residual viscosity was 260 mPa·s and the Casson yield value was 29479 mPa.

**[Table 5]**

| Ingredient | Content (% by mass) |
|---|---|
| (a) | |
| Squalane | 2 |
| White vaseline | 9 |
| Diisopropyl adipate | 4 |
| Cetanol | 1 |
| Glycerin monostearate | 2 |
| POE(60) hydrogenated castor oil | 1.5 |
| POE(23) cetyl ether | 2 |
| Medium chain fatty acid triglyceride | 6 |
| (b) | |
| 1,3-butanediol | 14 |
| Sodium edetate | 0.1 |
| Water | 55.5 |
| Carboxyvinyl polymer | 0.7 |
| (c) | |
| Diisopropanolamine | 0.1 |
| Water | 2.1 |

For the skin external preparation 2 and the skin external preparation of Comparative Example 2, viscosity was measured by a cone-plate viscometer (apparatus model name: RE-80R, name of manufacturing company: TOKI SANGYO Co., Ltd., conditions: rotor: 3°×R14, measurement temperature: 25°C, rotation frequency: 50 rpm, measurement time: 3 minutes). As a result, the skin external preparation 2 had a viscosity of 1828 mPa· s and the skin external preparation of Comparative Example 2 had a viscosity of 1248 mPa·s.

It is guessed from this that the skin external preparation 2 produced by the production method of the present invention shows good spread in spreading since the residual viscosity is low and manifests high stability in preservation and high coating film strength after application since the Casson yield value is high.

### Example 3

Pharmaceutical compositions 3, 4 were produced according to formulations described in Table 6 and the method described in Example 1, and Comparative Examples 3, 4 were produced according to formulations described in Table 7 and the method described in Comparative Example 1. A carboxyvinyl polymer which a 0.2% aqueous solution thereof shows a viscosity of 2500 to 30000 mPa·s at 20°C in a neutral region was used. pH of the pharmaceutical composition 3 and Comparative Example 3 was 5.0, and pH of the pharmaceutical composition 4 and Comparative Example 4 was 5.5. For the pharmaceutical compositions produced, the residual viscosity and the Casson yield value were measured according to the following methods. For the pharmaceutical compositions, shear stress (S) against a shear rate (D) of 2 to 200 (1/s) was measured using a cone-plate viscometer (apparatus model name: RE-80R, name of manufacturing company: TOKI SANGYO Co., Ltd., conditions: rotor: 3°×R14, measurement temperature: 32°C, rotation frequency: 1, 10, 100 rpm, measurement time: 3 minutes), and the residual viscosity was calculated from the square of a gradient a in the relational equation; √S = a√D+b (a and b are a coefficient) and the Casson yield value was calculated from the square of an intercept b thereof. The results are shown in Tables 6 and 7. It is guessed from this that the pharmaceutical composition produced by the production method of the present invention shows good spread in spreading since the residual viscosity is low and manifests high stability in preservation and high coating film strength after application since the Casson yield value is high, as compared with the pharmaceutical composition produced by the production method of Comparative Example though the formulation is the same.

For the pharmaceutical compositions 3 and 4, usability was confirmed to find that a transient stimulus by the composition was not felt even on sensitive skin.

In contrast, usability was confirmed for the pharmaceutical compositions of Comparative Examples 3 and 4 to find that a transient stimulus by the composition was manifested to sensitive skin.

**[Table 6]**

| | Ingredient name | Pharmaceuti cal composition 3 | Pharmaceuti cal composition 4 |
|---|---|---|---|
| | Clobetasone butyrate | 0.05 | 0.05 |
| | Diisopropyl adipate | 3.00 | 3.00 |
| | Synthetic squalane | 2.00 | 2.00 |
| | Medium chain fatty acid triglyceride | 6.00 | 6.00 |
| (a) | White vaseline | 10.00 | 10.00 |
| | Cetanol | 1.50 | 1.50 |
| | Glycerin monostearate | 0.50 | 0.50 |
| | POE hydrogenated castor oil 60 | 1.50 | 1.50 |
| | POE cetyl ether | 2.00 | 2.00 |
| (b) | 1,3-butylene glycol | 12.00 | 12.00 |
| | Sodium edetate | 0.15 | 0.15 |
| | Methyl parahydroxybenzoate | 0.10 | 0.10 |
| | Water | 20.20 | 20.20 |
| (c) | Carboxyvinyl polymer | 0.60 | 0.60 |
| | Sodium hyaluronate | 0.01 | 0.01 |
| | Water | 39.39 | 39.39 |
| (d) | Diisopropanolamine | 0.24 | 0.40 |
| | Water | 0.76 | 0.60 |
| | Total | 100.00 | 100.00 |
| | Residual viscosity (mPa·s) | 135 | 211 |
| | Casson yield value | 61770 | 79241 |
| | Correlation coefficient | 0.9670 | 0.9640 |

**[Table 7]**

| | Ingredient name | Comparative Example 3 | Comparative Example 4 |
|---|---|---|---|
| (a) | Clobetasone butyrate | 0.05 | 0.05 |
| | Diisopropyl adipate | 3.00 | 3.00 |
| | Synthetic squalane | 2.00 | 2.00 |
| | Medium chain fatty acid triglyceride | 6.00 | 6.00 |
| | White vaseline | 10.00 | 10.00 |
| | Cetanol | 1.50 | 1.50 |
| | Glycerin monostearate | 0.50 | 0.50 |
| | POE hydrogenated castor oil 60 | 1.50 | 1.50 |
| | POE cetyl ether | 2.00 | 2.00 |
| (b) | 1,3-butylene glycol | 12.00 | 12.00 |
| | Sodium edetate | 0.15 | 0.15 |
| | Methyl parahydroxybenzoate | 0.10 | 0.10 |
| | Carboxyvinyl polymer | 0.60 | 0.60 |
| | Sodium hyaluronate | 0.01 | 0.01 |
| | Water | 59.59 | 59.59 |
| (c) | Diisopropanolamine | 0.24 | 0.40 |
| | Water | 0.76 | 0.60 |
| | Total | 100.00 | 100.00 |
| | Residual viscosity (mPa·s) | 368 | 547 |
| | Casson yield value | 40442 | 27926 |
| | Correlation coefficient | 0.9960 | 0.9177 |

### Example 4

Skin external preparations of Examples and Comparative Examples were produced based on formulations shown in Tables 8 and 9. The skin external preparations of Examples were prepared by the same method as for the skin external preparation 1. In contrast, the skin external preparations of Comparative Examples 5 to 7 were prepared by the following method.

That is, (a), (b) and (c) were weighed, and of them, (a) and (b) were mixed and dissolved with stirring at 75°C and (c) was mixed and dissolved with stirring at room temperature, and preserved at respective dissolution temperatures. Under stirring, to (b) was added (a) gradually and emulsification was caused, and when a uniform emulsion was obtained, the emulsion was cooled with stirring, and when cooled down to 30°C, further, (c) was added gradually and neutralization was accomplished, to obtain a skin external preparation of Comparative Example. For any skin external preparation, the viscosity at 25°C was 1000 to 3000 mPa·s.

**[Table 8]**

| | Ingredient name | Skin external preparation 5 | Skin external preparation 6 | Skin external preparation 7 |
|---|---|---|---|---|
| | Clobetasone butyrate | 0.05 | 0.05 | 0.05 |
| | Diisopropyl adipate | 3.00 | 3.00 | 3.00 |
| | Synthetic squalane | 2.00 | 2.00 | 2.00 |
| | Medium chain fatty acid triglyceride | 6.00 | 6.00 | 6.00 |
| (a) | White vaseline | 10.00 | 10.00 | 10.00 |
| | Cetanol | 1.50 | 1.50 | 1.50 |
| | Glycerin monostearate | 0.50 | 0.50 | 0.50 |
| | Polyoxyethylene hydrogenated castor oil | 1.50 | 1.50 | 1.50 |
| | Polyoxyethylene cetyl ether | 2.00 | 2.00 | 2.00 |
| (b) | 1,3-butylene glycol | 12.00 | 12.00 | 12.00 |
| | Sodium edetate | 0.15 | 0.15 | 0.15 |
| | Methyl parahydroxybenzoate | 0.10 | 0.10 | 0.10 |
| | Water | 20.20 | 20.20 | 20.20 |
| (c) | Carboxyvinyl polymer | 0.60 | 0.60 | 0.60 |
| | Sodium hyaluronate | 0.01 | 0.01 | 0.01 |
| | Water | 39.39 | 39.39 | 39.39 |
| (d) | Diisopropanolamine | 0.08 | 0.24 | 0.4 |
| | Water | 0.92 | 0.76 | 0.6 |
| | Total | 100.00 | 100.00 | 100.0 |
| pH | | 4.5 | 5.0 | 5.5 |

**[Table 9]**

| | Ingredient name | Comparative Example 5 | Comparative Example 6 | Comparative Example 7 |
|---|---|---|---|---|
| | Clobetasone butyrate | 0.05 | 0.05 | 0.05 |
| | Diisopropyl adipate | 3.00 | 3.00 | 3.00 |
| | Synthetic squalane | 2.00 | 2.00 | 2.00 |
| | Medium chain fatty acid triglyceride | 6.00 | 6.00 | 6.00 |
| (a) | White vaseline | 10.00 | 10.00 | 10.00 |
| | Cetanol | 1.50 | 1.50 | 1.50 |
| | Glycerin monostearate | 0.50 | 0.50 | 0.50 |
| | Polyoxyethylene hydrogenated castor oil | 1.50 | 1.50 | 1.50 |
| | Polyoxyethylene cetyl ether | 2.00 | 2.00 | 2.00 |
| | 1,3-butylene glycol | 12.00 | 12.00 | 12.00 |
| | Sodium edetate | 0.15 | 0.15 | 0.15 |
| (b) | Methyl parahydroxybenzoate | 0.10 | 0.10 | 0.10 |
| | Carboxyvinyl polymer | 0.60 | 0.60 | 0.60 |
| | Sodium hyaluronate | 0.01 | 0.01 | 0.01 |
| | Water | 59.59 | 59.59 | 59.59 |
| (c) | Diisopropanolamine | 0.08 | 0.24 | 0.4 |
| | Water | 0.92 | 0.76 | 0.6 |
| | Total | 100.00 | 100.00 | 100.0 |
| pH | | 4.5 | 5.0 | 5.5 |

The spread meter diameters at 5°C, 25°C and 32°C were measured three times by the method described above, and the average and the standard deviation thereof were calculated. The results are shown in Table 10.

**[Table 10]**

| | | Spread meter diameter (mm) | | | | | |
|---|---|---|---|---|---|---|---|
| | | Skin external preparation 5 | Skin external preparation 6 | Skin external preparation 7 | Comparative Example 5 | Comparative Example 6 | Comparative Example 7 |
| 5°C | 1 | 39.34 | 36.10 | 35.10 | 42.75 | 36.34 | 33.79 |
| | 2 | 38.78 | 35.50 | 34.83 | 41.98 | 36.18 | 33.58 |
| | 3 | 39.11 | 35.42 | 34.78 | 42.58 | 36.29 | 33.87 |
| | Ave. | 39.08 | 35.67 | 34.90 | 42.44 | 36.27 | 33.75 |
| | SD | 0.28 | 0.37 | 0.17 | 0.4 | 0.08 | 0.15 |
| 25°C | 1 | 41.44 | 37.78 | 36.21 | 41.99 | 36.88 | 35.31 |
| | 2 | 40.12 | 37.88 | 36.11 | 42.28 | 37.21 | 35.61 |
| | 3 | 41.05 | 37.25 | 35.78 | 42.54 | 36.94 | 35.12 |
| | Ave. | 40.87 | 37.64 | 36.03 | 42.27 | 37.01 | 35.35 |
| | SD | 0. 68 | 0.34 | 0.23 | 0.28 | 0.18 | 0.25 |
| 32°C | 1 | 42.08 | 39.49 | 37.31 | 46.64 | 40.22 | 39.43 |
| | 2 | 42.78 | 39.05 | 37.21 | 46.87 | 40.75 | 39.58 |
| | 3 | 42.57 | 39.11 | 37.25 | 46.28 | 40.59 | 39.03 |
| | Ave. | 42.48 | 39.22 | 37.26 | 46.6 | 40.52 | 39.35 |
| | SD | 0.36 | 0.24 | 0.05 | 0.3 | 0.27 | 0.28 |
| 5-32°C variation range | Standard deviation of average value at each temperature | 1.7 | 1.78 | 1.18 | 2.45 | 2.27 | 2.88 |
| | Variance of average value at each temperature | 2.9 | 3.2 | 1.4 | 6 | 5.2 | 8.3 |

As shown in Table 10, all the skin external preparations of Examples showed a variance in a range of 1 to 4, while all the skin external preparations of Comparative Examples showed a variance larger than 4.

For the resultant skin external preparations of Examples and Comparative Examples, usability was evaluated by panelists having sensitive skin to find that the skin external preparations of Examples could be applied without feeling a transient stimulus and were excellent in a feeling of use. In contrast, the skin external preparations of Comparative Examples caused a transient stimulus.

Further, for the skin external preparation 6 and the skin external preparation of Comparative Example 6, a feeling of use was evaluated by three panelists having sensitive skin. The results are shown in Table 11.

**[Table 11]**

| Evaluation item | | | Skin external preparation 6 | Comparative Example 6 |
|---|---|---|---|---|
| | Spreadability | Very good | 2 | 1 |
| | | Good | 1 | 2 |
| | | Poor | | |
| | | Very poor | | |
| | Oiliness | Very good | 3 | |
| Feeling of use | | Good | | 2 |
| | | Bad | | 1 |
| | | Very bad | | |
| | Residual feeling | Very good | 2 | |
| | | Good | 1 | 3 |
| | | Bad | | |
| | | Very bad | | |

### Industrial Applicability

The present invention can be applied to a medicine.

## Claims

1) A method of producing an external composition of an oil-in-water emulsifier type containing a carboxyvinyl polymer optionally alkyl-modified and/or a salt thereof and a nonionic surfactant, wherein an oil-in-water emulsion is prepared at a temperature higher than the cloud point of the nonionic surfactant, the emulsion is cooled to a temperature not higher than the cloud point of the nonionic surfactant, then, a carboxyvinyl polymer is added, and thereafter, the carboxyvinyl polymer is neutralized with an alkaline agent.

2. The production method of the composition according to Claim 1, wherein the temperature higher than the cloud point is 75 to 90°C.

3. The production method according to Claim 1 or 2, wherein the temperature not higher than the cloud point is 25 to 35°C.

4. The production method of the composition according to any one of Claims 1 to 3, wherein the nonionic surfactant is selected from a polyoxyethylene alkyl ether, castor oil optionally polyoxyethylene-hardened and a fatty acid monoglyceride.

5. The production method of the composition according to any one of Claims 1 to 4, wherein the surfactant is only a nonionic surfactant.

6. The production method of the composition according to any one of Claims 1 to 5, wherein the composition contains one selected from a moisturizer, an antihistamine agent, a non-steroidal anti-inflammatory agent, a steroid, a bactericide, an anti-fungal agent, an antibiotic, an anti-virus agent, an immunosuppressive agent and vitamins, as an active ingredient.

7. A composition produced by the production method of the composition as described in any one of Claims 1 to 6 and comprising a carboxyvinyl polymer optionally alkyl-modified and/or a salt thereof and a nonionic surfactant, wherein the residual viscosity measured by a cone-plate viscometer under 32°C homeothermic condition is 200 mPa·s or less and the Casson yield value thereof is 40000 mPa or more.
